(19) Europäisches Patentamt
European Patent Office
Office européen des brevets

(11) EP 0 795 313 A2

(12) **EUROPÄISCHE PATENTANMELDUNG**

(43) Veröffentlichungstag:
**17.09.1997 Patentblatt 1997/38**

(51) Int. Cl.$^6$: **A61K 7/13**

(21) Anmeldenummer: **96119343.0**

(22) Anmeldetag: **03.12.1996**

(84) Benannte Vertragsstaaten:
**DE ES FR GB IT**

(30) Priorität: **16.03.1996 DE 19610392**

(71) Anmelder: **Wella Aktiengesellschaft
64274 Darmstadt (DE)**

(72) Erfinder:
- **Kunz, Manuela, Dr.
1723 Marly (CH)**
- **Le Cruer, Dominique
1729 Bonnefontaine (CH)**

(54) **Mittel und Verfahren zum oxidativen Färben von Keratinfasern**

(57) Gegenstand der Erfindung ist ein Mittel zum oxidativen Färben von Keratinfasern - insbesondere Haaren - auf der Basis einer Entwicklersubstanz-/Kupplersubstanz-Kombination und mit einem pH-Wert von 6 bis 9,5, welches dadurch gekennzeichnet ist, daß es ein Sauerstoff-Oxidoreductase/Substrat-System und eine Peroxidase sowie als Kupplersubstanz bestimmte m-Phenylendiaminderivate enthält.

EP 0 795 313 A2

**Beschreibung**

Die vorliegende Erfindung betrifft ein Mittel zum oxidativen Färben von Keratinfasern auf der Basis einer Entwicklersubstanz-/Kupplersubstanz-Kombination und eines enzymatizchen Oxidationssystems sowie ein Verfahren zum oxidativen Färben von Keratinfasern unter Verwendung dieses Mittels.

Auf dem Gebiet der Färbung von Keratinfasern haben oxidative Färbemittel eine wesentliche Bedeutung erlangt. Die Färbung entsteht hierbei durch Reaktion bestimmter Entwicklersubstanzen mit bestimmten Kupplersubstanzen in Gegenwart eines geeigneten Oxidationsmittels. Entwicklersubstanzen und Kupplersubstanzen werden normalerweise erst unmittelbar vor der Anwendung mit dem Oxidationsmittel vermischt. Die durch die Vermischung erhaltene Färbemasse wird sodann auf die zu färbenden Keratipfasern aufgetragen. Da überschüssige Färbemasse nicht aufbewahrt werden kann, muß die verbleibende restliche Färbemasse entsorgt werden. Normalerweise wird als Oxidationsmittel eine 2- bis 9%ige Wasserstoffperoxidzubereitung verwendet. Der Einsatz derart hoher Oxidationsmittelkonzentrationen kann, insbesondere bei gebleichtem oder dauergewelltem Haar, jedoch zu einer Schädigung der Haare führen.

Um zufriedenstellende Färbeergebnisse, die mehrere Haarwäschen überdauern, zu erzielen, erfolgt zudem die Haarfärbung normalerweise im stark alkalischen pH-Bereich (pH > 9), wodurch eine zusätzliche Schädigung der Haarfasern hervorgerufen wird.

Bei der Verwendung von Enzymen als Biokatalysatoren kann die Oxidation der Entwicklersubstanz-/Kupplersubstanz-Kombination hingegen mit wenig oder sogar ohne Wasserstoffperoxid erfolgen, wobei ein pH-Wert des Färbemittels im schwach sauren bis schwach alkalischen Bereich ausreichend ist. Hierdurch wird eine größere Schonung der Haarfaser ermöglicht.

Ein Nachteil von derartigen enzymatisch katalysierten oxidativen Färbungen ist die gegenüber üblichen oxidativen Färbungen geringere Intensität und Haltbarkeit der Färbung.

Auf einer enzymatischen Oxidation basierende Oxidationshaarfärbemittel sind beispielsweise aus der WO 94/00 100 sowie der EP-OS 0 310 675 bekannt. Die in diesen Veröffentlichungen beschriebenen Mittel weisen jedoch eine Reihe von Nachteilen auf. So erfordert das aus der WO 94/00 100 bekannte Färbeverfahren als Farbstoffvorstufe bestimmte Indole oder Indoline sowie eine mehrstufige Arbeitsweise, während mit den in der EP-OS 0 310 675 beschriebenen Färbemitteln keine befriedigenden Färbeergebnisse erzielt werden können, wobei insbesondere die Färbung von intensiven dunklen Farbtönen unzureichend ist.

Es bestand daher die Aufgabe, ein Mittel sowie ein Verfahren zur oxidativen Färbung von Keratinfasern, insbesondere menschlichen Haaren, zur Verfügung zu stellen, welches auf einfache Weise und bei größtmöglicher Schonung der Keratinfasern intensive und waschbeständige Ausfärbung, insbesondere auch im Naturtonbereich, ermöglicht.

Hierzu wurde nunmehr gefunden, daß ein Mittel zum oxidativen Färben von Keratinfasern - insbesondere Haaren - auf der Basis einer Entwicklersubstanz-/Kupplersubstanz-Kombination und mit einem pH-Wert von 6 bis 9,5, welches dadurch gekennzeichnet ist, daß es ein Sauerstoff-Oxidoreductase/Substrat-System und eine Peroxidase sowie als Kupplersubstanz ein m-Phenylendiamin der Formel (I) oder dessen physiologisch verträgliches Salz enthält,

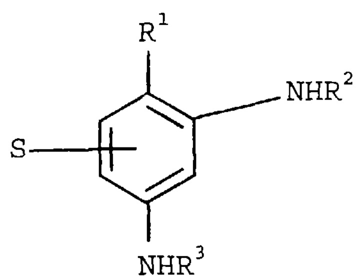

(I),

wobei

| | |
|---|---|
| $R^1$ | gleich einem $C_1$- bis $C_6$-Alkoxyrest, einem unsubstituierten oder mit einer oder mehreren Hydroxygruppen, Halogenatomen oder $C_1$- bis $C_6$-Alkoxygruppen substituierten $C_1$- bis $C_6$-Alkylrest ist; |
| $R^2$, $R^3$ | unabhängig voneinander gleich Wasserstoff oder einem unsubstituierten oder mit einer oder mehreren Hydroxygruppen, Halogenatomen oder $C_1$- bis $C_6$-Alkoxygruppen substituierten $C_1$- bis $C_6$-Alkylrest ist, wobei der Alkylrest gegebenenfalls durch maximal 2 Sauerstoffatome in Etherfunktion unterbrochen sein kann; und |
| S | gleich Wasserstoff oder einem $C_1$- bis $C_6$-Alkylrest ist; |

die gestellte Aufgabe in hervorragender Weise löst.

Besonders geeignete Verbindungen der Formel (I) sind

4-(2'-Hydroxyethyl)-1,3-diaminobenzol-Dihydrochlorid,
4-Propyl-1,3-diaminobenzol-Dihydrochlorid,
4-(2',2',2'-Trifluorethoxy)-1,3-diaminobenzol,
4-(2'-Hydroxyethoxy)-1,3-diaminobenzol-Dihydrochlorid,
2-Amino-4-methoxyethylamino-anisol,
2-Amino-4-hydroxypropylamino-anisol-Dihydrochlorid,
2-Amino-4-(2',2',2'-Trifluorethyl)amino-anisol,
4-Amino-2-ethylamino-anisol-Dihydrochlorid,
4-Amino-2-(2',2',2'-trifluorethyl)amino-anisol und
2-Amino-4-(2'-hydroxyethyl)amino-anisolsulfat.

Als Entwicklersubstanzen können aromatische Amine, Diaminophenole oder Aminophenole, deren Amino- oder Hydroxygruppen in ortho- oder para-Position zueinander stehen, beispielsweise 1,4-Diaminobenzol, 2,5-Diaminotoluol, 2,5-Diamino-phenylethanol, o-Aminophenol oder 4-Amino-m-kresol, genannt werden, wobei auch Mischungen dieser Entwicklersubstanzen verwendet werden können.

Obwohl eine Verwendung der Verbindungen der Formel (I) als alleinige Kupplersubstanz bevorzugt ist, ist es selbstverständlich möglich, die Kupplersubstanzen der Formel (I) gemeinsam mit bekannten Kupplersubstanzen, wie zum Beispiel aromatischen m-Diaminen, m-Aminophenolen, Polyphenolen oder Naphtholen, insbesondere 1,3-Diaminobenzol, m-Aminophenol, $\alpha$-Naphthol, Resorcin oder 3-Amino-6-methylphenol, einzusetzen.

Die Gesamtmenge der in dem hier beschriebenen Haarfärbemittel enthaltenen Entwicklersubstanz-Kupplersubstanz-Kombination soll etwa 0,1 bis 8 Gewichtsprozent, vorzugsweise etwa 0,5 bis 4 Gewichtsprozent, betragen.

Die Entwicklersubstanzen und Kupplersubstanzen werden im allgemeinen in äquimolaren Mengen eingesetzt. Es ist jedoch nicht nachteilig, wenn eine dieser beiden Verbindungen in einem gewissen Überschuß oder Unterschuß eingesetzt wird.

Je nach gewünschter Nuance können außerdem ein oder mehrere direktziehende Farbstoffe, insbesondere Nitrofarbstoffe, wie 4-Amino-3-nitrophenol, 2-Amino-6-chlor-4-nitrophenol, 1-Hydroxy-2-(2'-hydroxyethyl)amino-4,6-dinitrobenzol, 1-Amino-2-nitro-4-(2'-hydroxyethyl) amino-5-chlorbenzol und/oder 4-(3'-Hydroxypropyl)amino-3-nitrophenol zugesetzt werden.

Das Färbemittel kann diese Farbstoffe in einer Gesamtmenge von etwa 0,1 bis 4 Gewichtsprozent enthalten, wobei die Gesamtmenge des Farbstoffgemisches aus diesen Farbstoffen und der Entwicklersubstanz-/Kupplersubstanz-Kombination etwa 0,1 bis 8 Gewichtsprozent, vorzugsweise 0,5 bis 5 Gewichtsprozent, beträgt.

Das in dem erfindungsgemäßen Färbemittel verwendete Sauerstoff-Oxidoreductase/Substrat-System ermöglicht eine Oxidation der Entwicklersubstanz-/Kupplersubstanz-Kombination durch Luftsauerstoff ohne Wasserstoffperoxidzusatz, wobei insbesondere folgende Oxidoreductase/Substrat-Systeme eingesetzt werden können:

Glucose-Oxidase (EC 1.1.3.4)/D-Glucose
Alkohol-Oxidase (EC 1.1.3.13)/Ethanol
Pyruvat-Oxidase (EC 1.2.3.3)/Pyruvat
Oxalat-Oxidase (EC 1.2.3.4)/Oxalat
Cholesterin-Oxidase (EC 1.1.3.6)/Cholesterin
Uricase (EC 1.7.3.3)/Harnsäure
Lactat-Oxidase/Milchsäure
Xanthin-Oxidase (EC 1.1.3.22)/Xanthin

Besonders bevorzugt ist hierbei die Verwendung des Systems Glucose-Oxidase/D-Glucose, bei dem mit Luftsauerstoff und Wasser in situ Wasserstoffperoxid gebildet wird, in Kombination mit Peroxidase (EC 1.11.1.7). Sowohl die Glucose-Oxidase als auch die Peroxidase sind vorzugsweise katalasefrei (Glucose-Oxidase: Katalaseaktivität $< 1$ % der Gesamt-Glucose-Oxidase-Aktivität; Peroxidase: Katalaseaktivität $< 0{,}5$ % der Gesamt-Peroxidase-Aktivität).

Die Menge an Oxidoreductase beziehungsweise Peroxidase ist abhängig von der Reinheit des verwendeten Enzyms. Sie beträgt in der Regel für die Oxidoreductase - bezogen auf das Aktivitätsbestimmungssystem (beispielsweise für Glucose-Oxidase: Glucose/Gujacol/$O_2$-gesättigt; pH = 7; bei 25° C) 50 bis 20.000 Units pro 100 g Färbemittel, vorzugsweise 100 bis 1.000 Units pro 100 g Färbemittel, während die Peroxidase - bezogen auf das Aktivitätsbestimmungssystem (Gujacol/$H_2O_2$; pH = 7; 25° C) - in einer Menge von 50 bis 20.000 Units pro 100 g Färbemittel, vorzugsweise 100 bis 1.000 Units pro 100 g Färbemittel, eingesetzt wird.

Die Menge an Substrat beträgt in der Regel 0,5 bis 25 Gewichtsprozent, vorzugsweise 1 bis 15 Gewichtsprozent.

Darüber hinaus können in dem erfindungsgemäßen Mittel weitere übliche kosmetische Zusätze, wie zum Beispiel

Parfümöle, Konservierungsstoffe, Komplexbildner, Verdicker oder Pflegestoffe, enthalten sein.

Die Zubereitungsform des neuen Haarfärbemittels kann beispielsweise eine Lösung, insbesondere eine wäßrige oder wäßrig-alkoholische Lösung, eine Creme, ein Gel oder eine Emulsion sein. Ihre Zusammensetzung stellt eine Mischung der Farbstoffkomponenten mit dem Enzym/Substrat-System und für solche Zubereitungen üblichen Zusätzen dar.

Übliche Zusätze in Lösungen, Cremes, Emulsionen oder Gelen sind zum Beispiel Lösungsmittel wie Wasser, niedere aliphatische Alkohole, beispielsweise Ethanol, Propanol oder Isopropanol, Glycerin, Glykolether oder Glykole wie 1,2-Propylenglykol, weiterhin Netzmittel oder Emulgatoren aus den Klassen der anionischen, kationischen, amphoteren oder nichtionogenen oberflächenaktiven Substanzen wie Fettalkoholsulfate, oxethylierte Fettalkoholsulfate, Alkylsulfonate, Alkylbenzolsulfonate, Alkyltrimethylammoniumsalze, Alkylbetaine, oxethylierte Fettalkohole, oxethylierte Nonylphenole, Fettsäurealkanolamide, oxethylierte Fettsäureester, ferner Verdicker wie höhere Fettalkohole, Stärke, Cellulosederivate, Petrolatum, Paraffinöl und Fettsäuren sowie außerdem Pflegestoffe wie kationische Harze, Lanolinderivate, Cholesterin, Pantothensäure und Betain. Die erwähnten Bestandteile werden in den für solche Zwecke üblichen Mengen verwendet, zum Beispiel die Netzmittel und Emulgatoren in Konzentrationen von etwa 0,5 bis 30 Gewichtsprozent, die Verdicker in einer Menge von etwa 0,1 bis 25 Gewichtsprozent und die Pflegestoffe in einer Konzentration von etwa 0,1 bis 5,0 Gewichtsprozent.

Besonders vorteilhaft ist hierbei der Zusatz von nichtionischen Tensiden - insbesondere Fettalkoholpolyglykolethern der Formel (II)

$$R\text{-}O\text{-}(CH_2\text{-}CH_2\text{-}O)_n\text{-}H \qquad \text{(II)},$$

mit R gleich einem $C_1$- bis $C_{20}$-Alkylrest und n gleich einer ganzen Zahl von 2 bis 20 - in einer Menge von 0,1 bis 10 Gewichtsprozent.

Die erfindungsgemäßen Mittel können weiterhin Säuren oder Basen sowie ein Puffersystem zur Einstellung des pH-Wertes enthalten. Als Basen oder Alkalisierungsmittel kommen hierbei Alkanolamine, Alkylamine, Alkalihydroxide und Ammoniumhydroxide oder -carbonate in Betracht. Als Säuren können organische oder anorganische Säuren, wie zum Beispiel Milchsäure, Essigsäure, Weinsäure, Zitronensäure, Ascorbinsäure, Borsäure, Salzsäure oder Phosphorsäure, eingesetzt werden. Unter den geeigneten Puffersystemen können der Zitratpuffer, Phosphatpuffer oder Boratpuffer genannt werden, wobei der Boratpuffer (Borsäure/NaOH) und der Phosphatpuffer ($KH_2PO_4$/$K_2HPO_4$) im erfindungsgemäßen pH-Bereich von 6 bis 9,5 besonders geeignet ist.

Weiterhin kann das erfindungsgemäße Mittel in Form einer Aerosolzubereitung, beispielsweise als Aerosolschaum sowie in Form eines Färbepulvers oder -granulates vorliegen, wobei die Konfektionierung in Form eines Färbepulvers oder -granulates, welches eine Mischung der lyophilisierten Enzyme mit den übrigen pulverförmigen und möglichst wasserfreien Inhaltsstoffen darstellt und erst unmittelbar vor der Anwendung mit Wasser oder einem Wasser/Alkohol-Gemisch zur gebrauchsfertigen Färbezubereitung vermischt wird, besonders bevorzugt ist.

Ebenfalls ist es möglich, das erfindungsgemäße Mittel als Mehrkomponentenpräparat zu konfektionieren. So kann zum Beispiel das gebrauchsfertige Mittel unmittelbar vor der Anwendung durch Vermischen zweier Komponenten hergestellt werden, wobei die erste Komponente aus einem oder mehreren Enzymen (vorzugsweise in lyophilisierter Form) besteht, während die zweite Komponente die übrigen Bestandteile des Färbemittels enthält.

Weiterhin ist es möglich, das erfindungsgemäße Mittel in Form eines Dreikomponentenpräparates einzusetzen, wobei die erste Komponente aus einem oder mehreren Enzymen (vorzugsweise in lyophilisierter Form) besteht, während die zweite Komponente eine Mischung der übrigen pulverförmigen Inhaltsstoffe darstellt und als dritte Komponente Wasser oder ein Wasser/Alkohol-Gemisch verwendet wird.

Alternativ dazu kann bei Konfektionierung in Form eines Einkomponentenpräparates das Enzym auch in mikroverkapselter Form eingesetzt werden.

Bei der Anwendung des erfindungsgemäßen Färbemittels für die Haarfärbung wird eine für die Färbung der Haare ausreichende Menge, je nach Haarfülle etwa 60 bis 200 g, der Färbezubereitung auf die Haare aufgetragen.

Man läßt das Färbemittel bei 15 bis 50 °C je nach gewünschter Farbtiefe etwa 15 bis 60 Minuten lang, vorzugsweise 25 bis 55 Minuten lang, auf das Haar einwirken, spült sodann das Haar mit Wasser aus und trocknet es. Gegebenenfalls wird im Anschluß an diese Spülung mit einem Shampoo gewaschen. Abschließend wird das Haar getrocknet.

Die Einwirkungszeit des Haarfärbemittels kann deutlich verkürzt werden, wenn das erfindungsgemäße Haarfärbemittel in einen luftdichten Behälter (beispielsweise einer Aerosoldose) abgefüllt wird und vor dem Abfüllen unter Rühren einer 15- bis 60minütigen Voroxidation mit Luftsauerstoff unterzogen wird.

Das erfindungsgemäße Mittel zum Färben von Keratinfasern ist insbesondere für die Färbung menschlicher Haare geeignet und ermöglicht eine sehr haarschonende und äußerst intensive Haarfärbung, wobei insbesondere bei Zusatz von geeigneten direktziehenden Farbstoffen auch sehr dunkle Naturtöne erzielt werden können.

Die nachfolgenden Beispiele sollen den Gegenstand der Erfindung näher erläutern, ohne diesen hierauf zu beschränken.

## Beispiele

### Beispiel 1: Haarfärbemittel

| | |
|---|---|
| 0,025 mol | Entwicklersubstanz |
| 0,025 mol | Kupplersubstanz der Formel (I) |
| 400 Units | Glucose-Oxidase (EC 1.1.3.4) |
| 400 Units | Peroxidase (EC 1.11.1.7) |
| 5,0 g | Isopropanol |
| 2,0 g | 1,2-Propandiol |
| 1,4 g | Polyethylenglykol(20)stearylether |
| 1,0 g | Glycerin |
| 1,0 g | D-Glucose |
| 0,3 g | Dinatrium-ethylendiaminotetraessigsäure |
| 0,1 g | Ascorbinsäure |
| ad 100,0 g | Boratpuffer (0,1 molar) |

Der pH-Wert des Haarfärbemittels wird mit Natronlauge auf einen Wert von 7,7 eingestellt.

Das Haarfärbemittel wird auf gebleichtes Haar aufgetragen. Nach einer Einwirkungszeit von 30 beziehungsweise 60 Minuten bei Raumtemperatur (20 bis 25 °C) wird das Haar mit lauwarmem Wasser gespült und sodann getrocknet.

Die in Abhängigkeit von den verschiedenen Entwicklersubstanz-/Kupplersubstanz-Kombinationen erhaltenen Färbungen sind in der nachfolgenden Tabelle 1 zusammengefaßt.

Tabelle 1

| Bsp. | Entwicklersubstanz | Kupplersubstanz | Einwirkungszeit (Minuten) | Farbton Lab-Werte |
|---|---|---|---|---|
| 1a | Hydroxyethyl-p-phenylendiaminsulfat | 2-Amino-4-(2'-hydroxyethyl)-amino-anisolsulfat | | tiefblau |
| | | | 30 | L = 35,22 |
| | | | | a = 3,85 |
| | | | | b = -14,13 |
| | | | 60 | L = 25,08 |
| | | | | a = 4,94 |
| | | | | b = -12,42 |
| 1b | 2,5-Diaminotoluolsulfat | 2-Amino-4-(2'-hydroxyethyl)-amino-anisolsulfat | | tiefblau |
| | | | 30 | L = 30,94 |
| | | | | a = 4,06 |
| | | | | b = -13,94 |
| | | | 60 | L = 24,10 |
| | | | | a = 4,54 |
| | | | | b = -12,74 |
| 1c | 2,5-Diaminotoluolsulfat | 4-(2'-Hydroxyethoxy) -1,3-diaminobenzol-Dihydrochlorid | | tiefblau |
| | | | 30 | L = 32,67 |
| | | | | a = 3,55 |
| | | | | b = -15,85 |
| | | | 60 | L = 23,73 |
| | | | | a = 4,47 |
| | | | | b = -13,42 |
| 1d | 4-Amino-m-kresol | 2-Amino-4-(2'-hydroxyethyl)amino-anisolsulfat | | purpurrot |
| | | | 30 | L = 35,58 |
| | | | | a = 20,41 |
| | | | | b = -0,03 |
| | | | 60 | L = 27,80 |
| | | | | a = 18,02 |
| | | | | b = -0,11 |

**Beispiel 2: Haarfärbemittel**

| | |
|---|---|
| 0,025 mol | Entwicklersubstanz |
| 0,025 mol | Kupplersubstanz der Formel (I) |
| 400 Units | Glucose-Oxidase (EC 1.1.3.4) |
| 400 Units | Peroxidase (EC 1.11.1.7) |
| 5,000 g | Isopropanol |
| 2,000 g | 1,2-Propandiol |
| 1,400 g | Polyethylenglykol(20)stearylether |
| 1,000 g | Glycerin |
| 1,000 g | Glucose |
| 0,300 g | Dinatrium-ethylendiaminotetraacetat |
| 0,100 g | Ascorbinsäure |
| 0,075 g | 2-Amino-6-chlor-4-nitrophenol |
| ad 100,000 g | Boratpuffer (0,1 molar) |

Der pH-Wert des Haarfärbemittels wird mit Natronlauge auf einen Wert von 7,7 eingestellt. Die Anwendung des Haarfärbemittels erfolgt in der in Beispiel 1 angegebenen Weise. Die erhaltenen Färbeergebnisse sind in der Tabelle 2 zusammengefaßt.

Tabelle 2

| Bsp. | Entwicklersubstanz | Kupplersubstanz | Einwirkungszeit (Minuten) | Farbton Lab-Werte |
|---|---|---|---|---|
| 2a | Hydroxyethyl -p-phenylendiaminsulfat | 2-Amino-4-(2'-hydroxyethyl)-amino-anisolsulfat | 30<br><br><br>60 | braun<br>L = 32,57<br>a = 0,67<br>b = 4,71<br>L = 25,54<br>a = 0,89<br>b = 3,97 |
| 2b | 2,5-Diaminotoluolsulfat | 2-Amino-4-(2'-hydroxyethyl)-amino-anisolsulfat | 30<br><br><br>60 | braun<br>L = 31,11<br>a = 0,24<br>b = 2,67<br>L = 23,65<br>a = 0,84<br>b = 0,88 |
| 2c | 2,5-Diaminotoluolsulfat | 4-(2'-Hydroxyethoxy) -1,3-diaminobenzol-Dihydrochlorid | 30<br><br><br>60 | braun<br>L = 29,14<br>a = -0,26<br>b = 1,89<br>L = 21,24<br>a = 0,46<br>b = -0,40 |

**Beispiel 3: Pulverhaarfarbe**

| | |
|---|---|
| 0,520 g | 2-Amino-4-(2'-hydroxyethyl)amino-anisol-sulfat |
| 0,310 g | 4-Amino-3-methylphenol |
| 0,080 g | 4-Amino-2-hydroxytoluol |
| 0,075 g | 2-Amino-6-chloro-4-nitro-phenol |
| 400 Units | Glucose-Oxidase (EC 1.1.3.4) |
| 400 Units | Peroxidase (EC 1.11.1.7) |
| 1,000 g | D-Glucose |
| 0,400 g | Natriumhydroxid |
| 0,350 g | Hydroxyethylcellulose |
| 0,300 g | Dinatrium-ethylendiaminotetraacetat |
| 0,280 g | Borsäure |
| 0,100 g | Ascorbinsäure |

Die vorstehend aufgeführte Pulverhaarfarbe wird vor der Anwendung mit Wasser auf 100 g aufgefüllt und unter Schütteln oder Rühren vermischt.

Das so erhaltene Haarfärbemittel wird auf gebleichtes Haar aufgetragen. Nach einer Einwirkungszeit von 30 Minuten bei Raumtemperatur (20 - 25 °C) wird das Haar mit lauwarmem Wasser ausgespült und getrocknet.

Es wird eine intensive kupferfarbene Haarfärbung erhalten.

**Beispiel 4: Zweikomponenten-Haarfärbemittel**

| **Komponente A:** | |
|---|---|
| 0,025 mol | 2,5-Diamino-toluolsulfat |
| 0,025 mol | 2-Amino-4-(2'-hydroxyethyl)-amino-anisolsulfat |
| 5,000 g | Isopropanol |
| 2,000 g | 1,2-Propandiol |
| 1,400 g | Polyethylenglykol(20)stearylether |
| 1,000 g | Glycerin |
| 1,000 g | Glucose |
| 0,300 g | Dinatrium-ethylendiaminotetraacetat |
| 0,100 g | Ascorbinsäure |
| 0,075 g | 2-Amino-6-chlor-4-nitrophenol |
| ad 100,000 g | Boratpuffer (0,1 molar) |
| **Komponente B:** | |
| 400 Units | Glucose-Oxidase (EC 1.1.3.4) |
| 400 Units | Peroxidase (EC 1.11.1.7) |

Unmittelbar vor der Anwendung werden die Komponenten A und B miteinander vermischt und der pH-Wert mit Natronlauge auf 7,7 eingestellt.

Die Anwendung des so erhaltenen Haarfärbemittels erfolgt in der in Beispiel 1 beschriebenen Weise, wobei die Einwirkungszeit 30 Minuten beträgt.

Es wird eine intensive Braunfärbung der Haare erzielt.

Die in der vorliegenden Anmeldung verwendeten Prozentangaben stellen, soweit nicht anders vermerkt, Gewichtsprozente dar. Die Ermittlung der Lab-Farbmeßwerte erfolgte mit einem Farbmeßgerät der Firma Minolta, Typ Chromameter II. Die für die Enzyme in Klammern angegebenen Klassifizierungen erfolgten gemäß der "Classification of the International Union of Biochemistry on Nomenclature and Classification of Enzymes (1984)". Die Enzymkonzentrationen werden in der vorliegenden Anmeldung in "Units" - der von der Internationalen Union für Biochemie als Standardeinheit für alle Enzyme vorgeschlagenen Internationalen Einheit - angegeben.

**Patentansprüche**

**1.** Mittel zum oxidativen Färben von Keratinfasern auf der Basis einer Entwicklersubstanz-/Kupplersubstanz-Kombination und mit einem pH-Wert von 6 bis 9,5, welches dadurch gekennzeichnet ist, daß ein Sauerstoff-Oxidoreductase/Substrat-System und eine Peroxidase sowie als Kupplersubstanz ein m-Phenylendiamin der Formel (I)

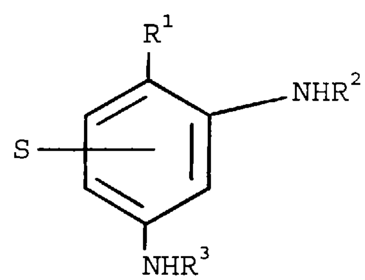

(I),

wobei

R1 gleich einem $C_1$- bis $C_6$-Alkoxyrest, einem unsubstituierten oder mit einer oder mehreren Hydroxygruppen, Halogenatomen oder $C_1$- bis $C_6$-Alkoxygruppen substituierten $C_1$- bis $C_6$-Alkylrest ist;

R2, R3 unabhängig voneinander gleich Wasserstoff oder einem unsubstituierten oder mit einer oder mehreren Hydroxygruppen, Halogenatomen oder $C_1$- bis $C_6$-Alkoxygruppen substituierten $C_1$- bis $C_6$-Alkylrest ist, wobei der Alkylrest gegebenenfalls durch maximal 2 Sauerstoffatome in Etherfunktion unterbrochen sein kann; und

S gleich Wasserstoff oder einem $C_1$- bis $C_6$-Alkylrest ist;

oder dessen physiologisch verträgliches Salz enthält.

**2.** Mittel nach Anspruch 1, dadurch gekennzeichnet, daß die Kupplersubstanz der Formel (I) ausgewählt ist aus 4-(2'-Hydroxyethyl)-1,3-diaminobenzol-Dihydrochlorid, 4-Propyl-1,3-diaminobenzol-Dihydrochlorid, 4-(2',2',2'-Trifluorethoxy)-1,3-diaminobenzol, 4-(2'-Hydroxyethoxy)-1,3-diaminobenzol-Dihydrochlorid, 2-Amino-4-methoxyethylaminoanisol, 2-Amino-4-hydroxypropylamino-anisol-Dihydrochlorid, 2-Amino-4-(2',2',2'-Trifluorethyl) amino-anisol, 4-Amino-2-ethylamino-anisol-Dihydrochlorid, 4-Amino-2-(2',2',2'-trifluorethyl)aminoanisol und 2-Amino-4-(2'-hydroxyethyl)aminoanisolsulfat.

**3.** Mittel nach Anspruch 1 oder 2, dadurch gekennzeichnet, daß die Entwicklersubstanz ausgewählt ist aus 1,4-Diaminobenzol, 2,5-Diaminotoluol, 2,5-Diaminophenylethanol, o-Aminophenol und 4-Amino-m-kresol.

**4.** Mittel nach einem der Ansprüche 1 bis 3, dadurch gekennzeichnet, daß die Gesamtmenge der enthaltenen Entwicklersubstanz-/Kupplersubstanz-Kombination 0,1 bis 8 Gewichtsprozent beträgt.

**5.** Mittel nach einem der Ansprüche 1 bis 4, dadurch gekennzeichnet, daß das Oxidoreductase/Substrat-System ausgewählt ist aus

Glucose-Oxidase (EC 1.1.3.4)/D-Glucose,
Alkohol-Oxidase (EC 1.1.3.13)/Ethanol,
Pyruvat-Oxidase (EC 1.2.3.3)/Pyruvat,
Oxalat-Oxidase (EC 1.2.3.4)/Oxalat,
Cholesterin-Oxidase (EC 1.1.3.6)/Cholesterin,
Uricase (EC 1.7.3.3)/Harnsäure,
Lactat-Oxidase/Milchsäure und
Xanthin-Oxidase (EC 1.1.3.22)/Xanthin.

**6.** Mittel nach einem der Ansprüche 1 bis 5, dadurch gekennzeichnet, daß die Oxidoreductase und die Peroxidase jeweils in einer Menge von 50 bis 20.000 Units/100 g Färbemittel eingesetzt wird.

**7.** Mittel nach einem der Ansprüche 1 bis 6, dadurch gekennzeichnet, daß das Substrat in einer Menge von 0,5 bis 25 Gewichtsprozent eingesetzt wird.

**8.** Mittel nach einem der Ansprüche 1 bis 7, dadurch gekennzeichnet, daß es einen oder mehrere direktziehende Farbstoffe aus der Gruppe bestehend aus 4-Amino-3-nitrophenol, 2-Amino-6-chlor-4-nitrophenol, 1-Hydroxy-2-(2'-hydroxyethyl)amino-4,6-dinitrobenzol, 1-Amino-2-nitro-4-(2'-hydroxyethyl) amino-5-chlorbenzol und 4-(3'-Hydroxypropyl)amino-3-nitrophenol enthält.

**9.** Mittel nach einem der Ansprüche 1 bis 8, dadurch gekennzeichnet, daß es 0,1 bis 10 Gewichtsprozent eines Fettalkoholpolyglykolethers der Formel (II)

$$R\text{-}O\text{-}(CH_2\text{-}CH_2\text{-}O)_n\text{-}H \qquad (II),$$

mit R gleich einem $C_1$- bis $C_{20}$-Alkylrest und n gleich einer ganzen Zahl von 2 bis 20, enthält.

**10.** Mittel nach einem der Ansprüche 1 bis 9, dadurch gekennzeichnet, daß es einen Zitrat-, Phosphat- oder Boratpuffer enthält.

**11.** Mittel nach einem der Ansprüche 1 bis 10, dadurch gekennzeichnet, daß es in Form einer wäßrigen oder wäßrig-alkoholischen Lösung, einer Creme, eines Geles, einer Emulsion, eines Aerosolschaumes, eines Pulvers oder eines Granulates vorliegt.

**12.** Mittel nach einem der Ansprüche 1 bis 11, dadurch gekennzeichnet, daß es ein Haarfärbemittel ist.

**13.** Mittel nach einem der Ansprüche 1 bis 12, dadurch gekennzeichnet, daß es in Form eines Mehrkomponentenpräparates konfektioniert ist.

**14.** Verfahren zum oxidativen Färben von Haaren, dadurch gekennzeichnet, daß man 60 bis 200 g eines Mittels gemäß einem der Ansprüche 1 bis 13 auf das Haar aufträgt und bei 15 bis 50 °C 15 bis 60 Minuten lang einwirken läßt, das Haar mit Wasser spült und sodann trocknet.

**15.** Verfahren nach Anspruch 14, dadurch gekennzeichnet, daß ein Färbepulver oder -granulat, welches vor der Anwendung mit Wasser zur gebrauchsfertigen Färbezubereitung vermischt wird, verwendet wird.